(19) Europäisches Patentamt / European Patent Office / Office européen des brevets

(11) **EP 3 963 325 B1**

(12) **EUROPEAN PATENT SPECIFICATION**

(45) Date of publication and mention of the grant of the patent:
**06.12.2023 Bulletin 2023/49**

(21) Application number: **20722569.9**

(22) Date of filing: **29.04.2020**

(51) International Patent Classification (IPC):
**G01N 33/543** (2006.01)

(52) Cooperative Patent Classification (CPC):
**G01N 33/54373**

(86) International application number:
**PCT/EP2020/061820**

(87) International publication number:
**WO 2020/221777 (05.11.2020 Gazette 2020/45)**

(54) **SELF-REFERENCED SENSOR**

SELBSTREFERENZIERTER SENSOR

CAPTEUR AUTORÉFÉRENCÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2019 EP 19382327**

(43) Date of publication of application:
**09.03.2022 Bulletin 2022/10**

(73) Proprietors:
- **Acondicionamiento Tarrasense (LEITAT)**
  **08225 Terrassa - Barcelona (ES)**
- **Universiteit Twente**
  **7522 NB Enschede (NL)**
- **Fundació Institut de Bioenginyeria de Catalunya (IBEC)**
  **08028 Barcelona (ES)**
- **Novelic Doo Beograd-Novi Beograd (Novelic)**
  **11000 Belgrade (RS)**

(72) Inventors:
- **MITJANS PRAT, Francesc**
  **08028 Barcelona (ES)**
- **PADILLA GARCÍA, Laura**
  **43882 Segur de Calafell - Tarragona (ES)**
- **KOZYREFF, Gregory**
  **B1030 Brussels (BE)**
- **ACHARYYA, Nirmalendu**
  **Hooghly, West Bengal PIN-712223 (IN)**
- **DE GOEDE, Michiel**
  **7523 BM Enschede (NL)**

- **CHANG, Lantian**
  **7521MC Enschede (NL)**
- **DIJKSTRA, Meindert**
  **7608 KV Almelo (NL)**
- **GARCIA BLANCO, Sonia M.**
  **7514JG Enschede (NL)**
- **RAMÓN AZCÓN, Javier**
  **08028 Barcelona (ES)**
- **OBREGÓN NÚÑEZ, Raquel**
  **08028 Barcelona (ES)**
- **MARTÍNEZ FRAIZ, Elena**
  **08028 Barcelona (ES)**
- **TOUDERT, Johann**
  **08860 Castelldefels - Barcelona (ES)**
- **MARTORELL PENA, Jordi**
  **08860 Castelldefels - Barcelona (ES)**
- **BRANKOVIC, Veselin**
  **11000 Belgrade (RS)**
- **MIHAJLOVIC, Veljko**
  **11000 Belgrade (RS)**
- **PARAUSIC, Marko**
  **11000 Belgrade (RS)**
- **TASOVAC, Darko**
  **11000 Belgrade (RS)**

(74) Representative: **ABG Intellectual Property Law, S.L.**
**Avenida de Burgos, 16D**
**Edificio Euromor**
**28036 Madrid (ES)**

(56) References cited:
**US-B1- 7 796 262**

EP 3 963 325 B1

- MUELLNER P ET AL: "Silicon photonic MZI sensor array employing on-chip wavelength multiplexing", OPTICAL AND QUANTUM ELECTRONICS, KLUWER ACADEMIC PUBLISHERS, BO, vol. 44, no. 12 - 13, 5 February 2012 (2012-02-05), pages 557-562, XP035119323, ISSN: 1572-817X, DOI: 10.1007/S11082-012-9557-0

**Description**

**TECHNICAL FIELD OF THE INVENTION**

The present invention relates to a label-free self-referenced sensor configured for sensing an analyte in a sample, using analyte receptors and non-invasive fluid sample analysis. More in particular, the present invention relates to a sensor that uses the principle of resonant micro rings that are operated by monitoring the induced resonance wavelength shift upon biomarkers binding to their surface.

**BACKGROUND OF THE INVENTION**

In medical diagnostics, there is an increasing demand for biosensors that can specifically detect biological analytes in a fluid, such as drug compounds, DNA oligomers, proteins and antibodies. The analytes are often only a few nanometers large, from few kDa down to few tens of Da in weight, can have concentrations down to the fg/ml-range and are typically present in a fluid that contains many other molecules at concentrations that are several orders of magnitude larger. Developing new sensing technologies is challenging.

Nowadays, biological research typically relies on the indirect detection of an analyte by attaching an easy to measure label to it, such as a fluorescent dye. However, this method often requires labelling strategies that typically involve multiple steps in the assay. It is therefore a very labour-intensive method. Therefore, label-free affinity biosensors have lately been receiving a lot of attention. They consist of a transducer with receptor molecules immobilized on its surface. In contrast to labelled detection methods, the transducer will respond directly to a selective affinity interaction between analyte molecules and the immobilized receptor molecules, allowing continuous and quantitative measurements. Ring resonators are good transducers for label-free biosensing, as they are highly manufacturable resonators of which the transmission spectrum heavily depends on the resonator's direct environment and that can be made with large quality factor, large extinction and low insertion loss. Further, they can be made very compact, allowing many of them to be incorporated on a single chip to perform simultaneous detection of multiple different analytes, along with measurements of their respective concentrations. They can also be made inexpensive when fabricated in high volumes with CMOS-compatible processes, so that the sensor chips can be disposable, meaning that the chip is only used once, avoiding complex cleaning of the sensor surface after use.

A ring resonator comprises a looped optical waveguide and a coupling mechanism to access the loop. When the waves in the loop build up a round trip phase shift that equals an integer times $2\pi$, the waves interfere constructively and the resonator is in resonance. Prior to the measurement, analyte receptors that are selective to the analyte are immobilized on the resonator by chemically modifying its surface. First an aqueous buffer solution is flown over the sensor to determine the reference resonance wavelengths that are proportional to the effective roundtrip length of the resonator. Then the test solution is flown over the sensor, allowing analyte molecules to specifically bind to the immobilized receptors. The resulting change of the effective roundtrip length causes an increase of each resonance wavelength proportional to the number of binding events. By scanning the transmission spectrum of the resonator repeatedly with a tunable laser and measuring the resonance wavelength shift as a function of time, the concentration of the analyte and kinetic information about the binding of the analyte to the receptor can be determined. To eliminate the effects of bulk refractive index variations and temperature, a second "reference" ring should in principle be utilized, which is exposed to the analyte but blocked by a "passivation agent" avoiding unspecific protein adsorption.

The necessity to use a tunable laser and/or the use of spectrometer in order to monitor shifts in resonance frequency associated to analyte binding makes the usual method of operation very costly. Indeed, a tunable laser and a spectrum analyser with sufficient wavelength resolution are expensive apparatus. For this reason, this method of detection stays confined in specialized laboratory and cannot be brought to the point of care (see Sasi Mudumba et al., "Photonic ring resonance is a versatile platform for performing multiplex immunoassays in real time", Journal of Immunological Methods, 2017, vol 448, pp.34-43, https://doi.org/10.1016/j.jim.2017.05.005).

As a response to this issue, He et al. (He, Lina, et al. "Detecting single viruses and nanoparticles using whispering gallery microlasers." Nature nanotechnology 6.7 (2011): 428.) devised a microring laser on which nanoparticles could attach. Starting from a pristine lasing resonator, the binding of a particle on the rim of the resonator causes the lasing frequency to split into two, closely separated, frequencies. The combination of the two outputs at neighbouring frequencies leads to a beating signal in the radio-frequency range, which can be recorded with off-the-shelf electronic devices. This may potentially lower the cost of operation and render the sensor portable. However, further deposition of particles on the surface of the resonator changes the beating frequency in an unpredictable manner, sometimes increasing it, sometimes decreasing it. Therefore, the above method is not suited to determine concentration of analyte in the environment of the sensor.

US7796262 discloses a sensor and corresponding system with reader comprising at least one whispering gallery mode resonator, wherein the resonator is selectively functionalized for the attachment of analyte receptors. It does not comprise a Bragg grating arranged over at least a portion of the perimeter of the resonator.

MUELLNER P ET AL: "Silicon photonic MZI sensor array employing on-chip wavelength multiplexing", OPTICAL AND QUANTUM ELECTRONICS, KLUWER ACADEMIC PUBLISHERS, BO, vol. 44, no. 12 - 13, 5 February 2012, pages 557-562. discloses a combination of Bragg grating and a WGM resonator in a biosensor. The Bragg grating is not arranged over at least a portion of the perimeter of the resonator, but rather arranged separate therefrom in series.

## SUMMARY OF THE INVENTION

The present invention provides a solution for the aforementioned problems, by a sensor according to claim 1 and a sensing system according to claim 14. Preferred embodiments are defined in the dependent claims.

In a first inventive aspect a sensor comprises at least one whispering gallery mode resonator, wherein the resonator comprises a Bragg grating arranged over at least a portion of the perimeter of the resonator and wherein the resonator is selectively functionalized for the attachment of analyte receptors.

Along the description, the term analyte is used in a general sense to refer to a substance that is subject of an analysis and that, in the present invention, will be embodied as a molecule that can be attached to the resonators. In a preferred embodiment the analytes are biomarkers. In a more preferred embodiment the analytes are biomarkers characteristic of at least one type of cancer. Throughout this document a biomarker should be understood as a distinctive biological or biologically derived indicator of a process, event, or condition.

As stated in the Background of the invention, the splitting of the laser mode can be detected as a beatnote by a fast photodetector and RF (radiofrequency) electronics, from the MHz till the few tens of GHz. One main disadvantage of prior art biosensors using resonators to detect changes in the beating frequency is that, as further particles attach to the resonator, the frequency splitting can either decrease or increase, in an unpredictable manner. Hence, the prior art sensors can detect single binding events but they cannot infer the concentration.

Advantageously, in a microresonator with a Bragg grating, resonance splitting increases monotonously with the number of binding events, thus being indicative of analyte concentration.

Therefore, the application of a Bragg grating makes the sensor self-referenced in the sense that any temperature or ambient disturbance to the sensor (eg., variation of the bulk refractive index) will not induce any response on the sensor, reducing the noise level and therefore decreasing the limit of detection.

A Bragg grating is a periodic perturbation of the guided structure, which results in a periodic perturbation of the effective refractive index $n_{eff}$ of the guided mode. Due to the periodic perturbation, a certain amount of the light in the forward propagating mode is coupled into the backwards propagating mode or in other words reflected. For a certain periodic modulation of the medium, there is the possibility for constructive interference for certain wavelengths.

In particular, Bragg gratings alter the dispersion relation linking the propagation constant, $\beta$, and the vacuum wavenumber $k_0 = 2\pi/\lambda$ or frequency $v = c/\lambda$. The eigenmodes of a micro-ring are labelled by the integral orbital number $\ell$ and form a discrete set. To the value $\ell$ corresponds the local propagation constant $\beta = \ell/R$, R being the radius of the micro-ring.

The Bragg grating is achieved by a $\theta$-periodic modulation of the effective refractive index of the ring ($\theta$: azimuthal angle). The period of the grating along the perimeter is d. At the critical wavenumber $\beta c = \pi/d$, the unperturbed dispersion relation splits. For Bragg gratings of deep modulations, a gap in the frequency spectrum opens and the dispersion curve levels off.

The opening of the gap is proportional to the depth of the modulation of the refractive index associated to the grating. For shallower modulations, mode splitting, rather than a full gap, appears. In either cases, the splitting in frequency or the opening of the gap in the frequency domain is proportional to the depth of the refractive index modulation. As this depth of modulation is associated to the binding of analyte, the sensor of the present invention allows monitoring the modification of the spectrum and thus monitoring the binding of analyte. In the case of a small enough frequency splitting, the associated beating can be in the GHz range and, hence, recorded by conventional electronics.

In an embodiment the sensor comprises analyte receptors immobilized on the resonator. In a preferred embodiment the analyte receptors are configured for the binding of at least one biomarker. In a more preferred embodiment the analyte receptors are configured for the binding of at least one biomarker characteristic of at least one type of cancer, preferably renal, prostate and/or bladder cancer. In an embodiment the analyte receptors comprise antibodies.

In an embodiment, the analyte receptors immobilized on the resonator are antibodies configured for the binding of one of the following biomarkers:

- PSA, for Prostate cancer
- Engrailed 2, for Prostate cancer
- MSMbeta for Prostate cancer
- HAS-1, for Bladder cancer:
- HAS-2, for Bladder cancer:

- HYAL-3, for Bladder cancer:
- Cytokeratin 20 (CK20), for Bladder cancer:
- CAIX, for Renal cancer:
- Aquaporin1, Renal cancer:
- Adipophilin, Renal cancer:
- IL8, for Bladder cancer
- MMP9, for Bladder cancer
- MMP10, for Bladder cancer
- SERPINA1, for Bladder cancer
- VEGFA, for Bladder cancer
- ANG, for Bladder cancer
- CA9, for Bladder cancer
- APOE, for Bladder cancer
- SDC1, for Bladder cancer
- SERPINE1, for Bladder cancer
- HYAL1, for Bladder cancer
- CXCL 1, for Bladder cancer
- CXCR7, for Bladder cancer
- SDF1beta, for Bladder cancer

In another embodiment, the analyte receptors immobilized on the resonator are antibodies configured for the binding of one of the following biomarkers:

- DNA

- C-peptide

- Insuline

- CRP

In an embodiment the resonator is made of a first material and the grating is made of a second material different to the first material. Thus, according to this embodiment the grating can first be imprinted on the resonator in a material such as $SiO_2$, or polymer material, different to the resonator material, and then either the resonator surface or the grating surface is selectively functionalized for the subsequent attachment of analyte receptors, such as antibodies. The resonator may be functionalized such that the analyte receptors cover the full resonator or only a part of it.

In an embodiment, the resonator is made of at least a material selected from $Al_2O_3$, $Si_3N_4$, $SiO_2$, SiON, $TiO_2$, $Ta_2O_5$, $Te_2O_5$, phosphate glass, $KY(WO_4)_2$, YAG, ZBLAN.

In an embodiment, the grating is made of a material comprising at least one polymer material such as PMMA, $SiO_2$, SiOn, SiN, $TiO_2$.

In an embodiment the grating is a grating of analyte receptors directly arranged over a surface of the resonator. In this embodiment the resonator surface is selectively functionalized such that the analyte receptors attach onto the resonator surface in the form of a grating, wherein the grating extends over the whole resonator or over only a part of it.

A circular cavity with large radius of curvature can be adequately modelled as a slab waveguide with effective refractive index that provides confinement in the x-direction for waves propagating in the z-direction. Given the radius R of the cavity, the waveguide closes to itself after a distance equal to the perimeter of the cavity. Hence, the electromagnetic wave is periodic in z with period $L=2\pi R$.

The Bragg grating consists of a small periodic modulation of the effective refractive index in regions of the waveguide. The period of each modulated section is d and the grating contains N periods. The maximum number of period is N_max=L/d in which case the grating covers the full perimeter of the cavity.

Alternatively, or complementarily to a Bragg structure covering the whole perimeter of the cavity, one may cover only a fraction of the ring perimeter choosing the period d so that the mode of interest $\ell$ is such that $\beta = \ell /R$ is at some distance from the critical value $\beta c=\pi/d$. In this way the Bragg modulation induces only a small mode splitting for the value $\ell$ of interest. In an embodiment, the period of the grating corresponds substantially to said critical value this is, half the operating wavelength.

By properly patterning the ring with an analyte receptor, such as a biomarker ligant, it is possible to let the refractive index modulation, and hence the mode splitting, depend on the biomarker concentration.

One advantage of this is that only a small number N of modulation periods are necessary. In a simulation carried out, with data:

- R=200µm,
- Refractive index of the environment: n1=1.33 (water),
- Refractive index in the ring: square wave modulation between n2=1.65 and n3=1.635,
- modulation period d=0.305µm,

it was determined that only N=10 modulation periods were necessary, whereas N=4120 would be needed for a full coverage.

Advantageously, using a small number of modulation periods the duration and complexity of the grating fabrication is reduced.

In an embodiment, the resonator material is doped with a material providing laser gain, preferably a rare-earth material, a semiconductor material, or a transition metal ion doped material.

In an embodiment, the resonator is a ring resonator embodied as a looped optical waveguide and the sensor further comprises a coupling mechanism to access the looped optical waveguide. Throughout this document "ring" and micro ring are used as synonyms when referring to a ring resonator.

In another embodiment, the resonator is a shaped as a disk and the sensor further comprises a coupling mechanism to access the disk.

Preferably, the coupling mechanism comprises an optical waveguide positioned to achieve optical coupling with the looped optical waveguide or the disk.

In an embodiment, the looped optical waveguide has a circular shape.

In an embodiment, the resonator has a closed loop configuration comprising a plurality of sections, each section being configured for coupling with an optical waveguide at a wavelength. A ring designed according to this embodiment allows to design independently the amount of coupling of pump and signal. Further, the length of the ring can also be tuned without affecting the coupling region.

In an embodiment the full core of the resonator is made of $Al_2O_3$. In this embodiment, and in contrast with the prior art, the sensor is not based on a resonator covered with $Al_2O_3$ but the full core is made of $Al_2O_3$. Advantageously, lower loss waveguides are achieved in this embodiment, which translates in higher Q-factors. $Al_2O_3$ can also be effectively doped with rare earth ions, which converts the resonator into a resonator laser. Also, applying gain to a resonator, increases the Q-factor. Upon lasing, further increase of the Q-factor can lead to a reduction of the limit of detection. Upon binding events on the surface of the active resonator, a shift in the emission wavelength can be detected.

In an embodiment the resonator is fabricated in rare-earth ion doped $Al_2O_3$, wherein the rare-earth ions provide emission outside the absorption bands of water, the rare-earth ions being preferably selected from the group consisting of $Yb^{3+}$, $Nd^{3+}$, $Er^{3+}$, $Tm^{3+}$, and $Ho^{3+}$.

The resonator can be used either as a passive device or as an active device. If operated as a passive device, during the operation of the sensor the resonator is excited by a laser with a spectral bandwidth that is large enough to include the two split resonances. The two resonances are then extracted from the output of the resonator and combined to form a beating signal.

In the active configuration, the resonator material is doped with a gain medium and is capable of operating as a laser. Then, operating as a laser, the resonator emits one or several frequencies that are associated to resonator modes within the gain spectrum of the doping material.

In an embodiment the resonator is functionalized with heptane with a carboxylic acid terminated phosphonic acid.

In an embodiment the sensor comprises a plurality of resonators and a plurality of chambers, wherein the resonators are arranged such that each chamber comprises one resonator, wherein the sensor comprises a plurality of openings, each opening providing fluid communication between two adjacent chambers, and wherein the openings have different size.

In a second inventive aspect a sensing system comprises:

a sensor according to any of the embodiments according to the first inventive aspect, and
a readout unit configured to receive the sensor and comprising aligning means for aligning the sensor.

In an embodiment the sensing system comprises a laser configured to optically pump the at least one resonator when the sensor is received in the readout unit, a photodetector and processing electronics.

All the features described in this specification (including the claims, description and drawings) and/or all the steps of the described method can be combined in any combination, with the exception of combinations of such mutually exclusive features and/or steps.

## BRIEF DESCRIPTION OF THE DRAWINGS

To complete the description and provide for better understanding of the invention, a set of drawings is provided. Said drawings illustrate a preferred embodiment of the invention, which should not be interpreted as restricting the scope of the invention, but just as an example of how the invention can be carried out.

Figure 1 shows the spectra of a ring resonator with a grating.

Figure 2 shows $|\Psi_{lc+1} \pm \Psi_{lc-1}|^2$ as a function of $\theta$. The step function represents the distribution of molecules along the perimeter.

Figure 3 schematically shows the degeneracy lifting of the spectra when analyte molecules are attached to part of the grating.

Figure 4 shows the different responses upon biomarker detection of the passive and active embodiments of the biosensor according to the invention.

Figure 5 shows experimental results on mode splitting of a self-referenced sensor according to the invention.

Figure 6 shows experimental results on mode splitting of a self-referenced sensor according to the invention.

Figure 7 shows the mode-splitting profile for the grating, along with the sensitivity of the sensor for different biomarker concentrations.

Figure 8 schematically shows a sensor according to an embodiment of the present invention.

Figure 9 schematically shows an embodiment wherein the grating is generated by adding to the resonator a layer of a different material on a top surface of the resonator.

Figure 10 schematically shows an embodiment wherein the grating is fabricated by directly imprinting the antibodies onto the top surface of the resonator.

Figure 11 schematically shows a sensor according to an embodiment of the present invention. In this embodiment the micro ring resonator has a grating on the sidewall over a part of its perimeter.

Figure 12 shows a sensor according to an embodiment of the invention.

Figure 13 shows a readout scheme for an active resonator according to the invention.

Figure 14 shows an embodiment of a sensing system according to an embodiment of the invention.

Figure 15 shows a flowchart of an embodiment of a method of operation of the invention.

Figure 16 shows a flowchart of an embodiment of a method of operation of the invention.

Figure 17 shows an embodiment of the sensor wherein the resonator is shaped as a non-circular loop.

## DETAILED DESCRIPTION OF THE INVENTION

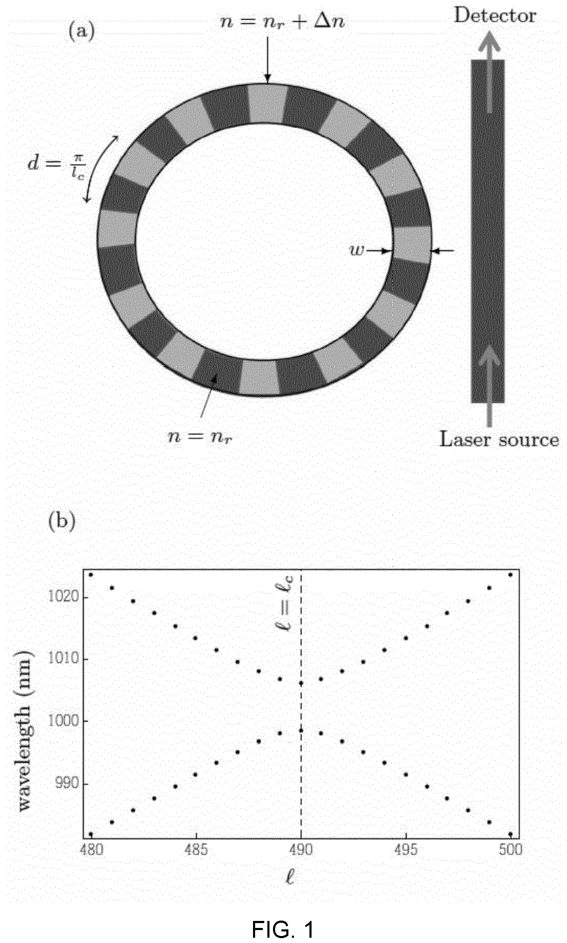

Figure 1 (a) schematically shows a microring resonator of width $\omega$ and radius R with a grating. The refractive index varies periodically between $n_r$ and $n_r + \Delta n$ with a period $d = \dfrac{\pi}{l_c}$ .

A random surface roughness eventually results in changing the refractive index of the ring from $n$ to $n + \Delta n(\theta)$. Being periodic, one may decompose the fluctuating index as

$$\Delta n(\theta) = \sum_{l=0}^{\infty} c_l \cos l\theta + s_l \sin l\theta$$

In the most random case, where $\Delta n(\theta)$ is a white noise, all Fourier coefficients appearing above have the same modulus. However, in practice, it may be that some Fourier coefficients are significantly bigger than other. This would be expected if the process that induces surface roughness has some systematic component with well-defined length scale.

Here, we assume that there is a grating in the ring, which results in an effective modulation $\Delta n(\theta)$ of the refractive index given by

$$\Delta n(\theta) = \delta \cos(2l_c \theta) + \cdots$$

Above, the omitted terms are higher harmonics in the Fourier decomposition of $\Delta n(\theta)$ and $\delta$ is small compared to 1 so that in can be treated as a perturbation. The modulated refractive index plays with the electromagnetic field an analogous role as a periodic potential does for electrons in a crystal. Hence this results in a band gap in the frequency spectrum near the edge of the Brillouin zone, $l = l_c$.

On the lower band of frequencies, the spectrum is given near $l = l_c$ by

$$\omega(l) \sim \omega_o(l_c) - \left[\Delta\omega + k\frac{(l - l_c)^2}{2}\right]$$

where $\Delta\omega = 0(\delta)$ is the frequency gap.

The corresponding eigenmode are now given by

$$\Psi_l \approx \Phi_l + \Phi_{l-2lc}$$

where $\Phi_m$ is an eigenmode of azimuthal number m of the resonator in the absence of refractive index modulation, that is when $\Delta n(\theta) = 0$.

Similarly, on the upper side of the band gap, the spectrum is given by

$$\omega(l) \sim \omega_o(l_c) + \left[\Delta\omega + k\frac{(l - l_c)^2}{2}\right]$$

with the corresponding eigenmodes approximately given by

$$X_l \approx \Phi_l - \Phi_{l-2lc}$$

Focusing now on the lower band, the modes with labels $\Psi_{lc+p}$ and $\Psi_{lc-p}$ are degenerate, as shown in Figure 1 (b). Therefore, any combination $a\,\Psi_{lc+p} + b\,\Psi_{lc-p}$ forms an eigenmode with approximate frequency $\omega(l_c + p)$ given by

$$\omega(l_c + p) \sim \omega_o(l_c) - \left[\Delta\omega + k\frac{p^2}{2}\right]$$

Figure 1 (b) shows the spectra of a ring resonator of width $\omega$ and radius R with a grating, where the refractive index varies periodically between $n_r$ and $n_r + \Delta n$, as shown in Figure 1(a). Here, $R = 50\mu m,\ \omega = 700nm,\ n_r = 1.65,\ \Delta n = -0.02$ and $l_c = 490$. The upper branch of resonances corresponds to eigenmodes of the form $X_l$, while the lower branch corresponds to $\Psi_l$.

For modelling a microring as the ones used in the embodiments of the present invention, a cavity mode is considered, with electric field ε, magnetic field H and frequency $\omega$. If the cavity is perturbed by a material with distributed polarizability $\alpha$, a polarization

$$P = \alpha \, \mathcal{E}$$

will result. Then, a frequency shift $\delta\omega$ and wavelength shift $\delta\lambda$ will be produced according to the formula

$$-\frac{\delta\omega}{\omega} = \frac{\delta\lambda}{\lambda} = \frac{\iiint \mathcal{E}^* . \alpha \, \mathcal{E} dV}{\iiint (\epsilon|\mathcal{E}|^2 + \mu|\mathrm{H}|^2) \, dV}$$

For assessing the frequency shift, the following integral has been used

$$U = \iiint \mathcal{E}^* . \alpha \, \mathcal{E} dV$$

In a particular embodiment, urine has been flown only over one quarter of the ring perimeter, in particular in the range $3\pi/4 < \theta < 5\pi/4$. Then, the eigenmodes

$$\Psi_{lc+1} + \Psi_{lc-1} \text{ and } \Psi_{lc+1} - \Psi_{lc-1}$$

experience the maximal and minimal frequency shift, respectively. These two particular combinations are the new eigenmodes in the presence of the urine polarisation P and their frequency difference ($U_+$ - $U_-$) is the mode splitting.

In order to derive the this result, we consider a pristine microring without grating, with a perfectly smooth surface, whose azimuthal dependence is characterized by their angular number $l$:

$$\Phi \equiv \begin{Bmatrix} \mathrm{E}_l(r,z) \\ \mathrm{H}_l(r,z) \end{Bmatrix} e^{il\theta - i\omega_0(l)t}$$

This equation can be simplified in the limit of large $l_c$, with $l = l_c \pm p$ and $p = O(1)$. Indeed, in that limit,

$$\begin{Bmatrix} \mathrm{E}_{\pm l}(r,z) \\ \mathrm{H}_{\pm l}(r,z) \end{Bmatrix} \sim \begin{Bmatrix} \mathrm{E}_{lc}(r,z) \\ \mathrm{H}_{lc}(r,z) \end{Bmatrix}$$

and, consequently,

$$\Psi_l \sim \begin{Bmatrix} \mathrm{E}_{lc}(r,z) \\ \mathrm{H}_{lc}(r,z) \end{Bmatrix} (e^{il\theta} + e^{i(l-2lc)\theta}) e^{-i\omega(l)t}$$

$$\rightarrow \Psi_{l+p} \sim \begin{Bmatrix} \mathrm{E}_{lc}(r,z) \\ \mathrm{H}_{lc}(r,z) \end{Bmatrix} e^{ip\theta} 2 \cos(l_c\theta) \, e^{-i\omega(l)t}$$

Hence, with a field given by

$$\Psi_{lc+1} + \Psi_{lc-1} \sim \begin{Bmatrix} \mathrm{E}_{lc}(r,z) \\ \mathrm{H}_{lc}(r,z) \end{Bmatrix} 4 \cos(l_c\theta) \cos(\theta) \, e^{-i\omega(l)t}$$

We find

$$U = U_+ = 16 \iint \mathrm{E}_{l_c}^*(r,z) \cdot \alpha \mathrm{E}_{l_c}(r,z) r \mathrm{d}r \mathrm{d}z \times \int_{\frac{3\pi}{4}}^{\frac{5\pi}{4}} \cos^2(l_c\theta) \cos^2(\theta)\,\mathrm{d}\theta$$

$$\rightarrow 2(2+\pi) \iint \mathrm{E}_{l_c}^*(r,z) \cdot \alpha \mathrm{E}_{l_c}(r,z) r \mathrm{d}r \mathrm{d}z$$

On the other hand, with

$$\Psi_{lc+1} - \Psi_{lc-1} \sim \begin{Bmatrix} \mathrm{E}_{lc}(r,z) \\ \mathrm{H}_{lc}(r,z) \end{Bmatrix} 4\,i\sin(l_c\theta)\cos(\theta)\,e^{-i\omega(l)t}$$

We obtain

$$U = U_- = -16 \iint \mathrm{E}_{l_c}^*(r,z) \cdot \alpha \mathrm{E}_{l_c}(r,z) r \mathrm{d}r \mathrm{d}z \times \int_{3\pi/4}^{5\pi/4} \cos^2(l_c\theta) \sin^2(\theta)\,\mathrm{d}\theta$$

$$\rightarrow 2(2-\pi) \iint \mathrm{E}_{l_c}^*(r,z) \cdot \alpha \mathrm{E}_{l_c}(r,z) r \mathrm{d}r \mathrm{d}z$$

The difference is

$$U_+ - U_- = 4\pi \iint \mathrm{E}_{l_c}^*(r,z) \cdot \alpha \mathrm{E}_{l_c}(r,z) r \mathrm{d}r \mathrm{d}z$$

Eventually the splitting is given by

$$\frac{\delta\lambda_+ - \delta\lambda_-}{\lambda} = \frac{2 \iint \mathrm{E}_{l_c}^*(r,z) \cdot \alpha \mathrm{E}_{l_c}(r,z) r \mathrm{d}r \mathrm{d}z}{\iiint \left(\epsilon |\mathrm{E}_{l_c}(r,z)|^2 + \mu |\mathrm{H}_{l_c}(r,z)|^2\right) r \mathrm{d}r \mathrm{d}z}$$

To illustrate the difference between $U_+$ and $U_-$, one may simply plot the square modulus of $\Psi_{lc+1} \pm \Psi_{lc-1}$ along the perimeter and compare it with the distribution of $\alpha$ along that perimeter.

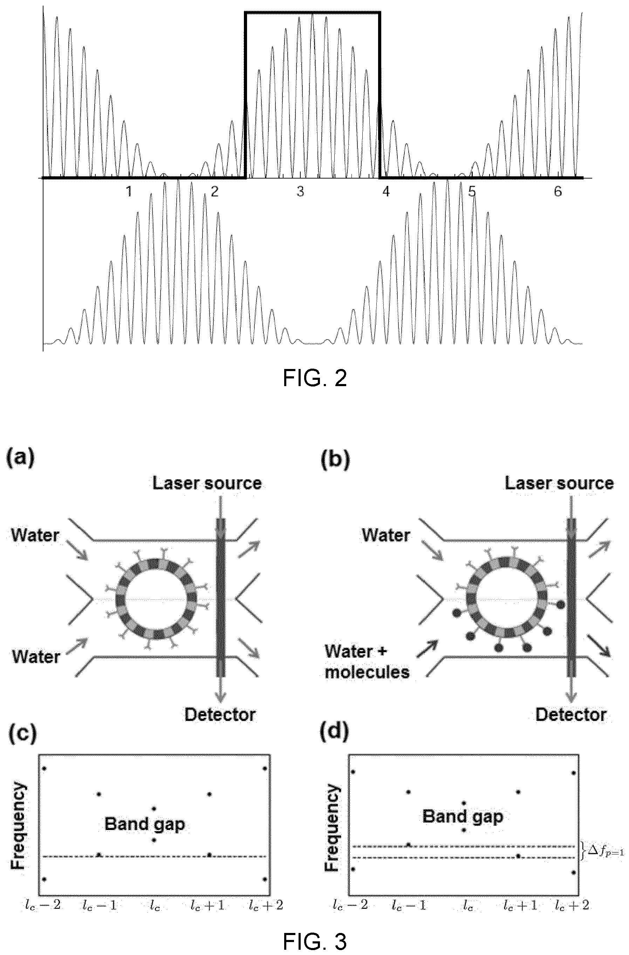

Said difference is schematically shown in Figure 2.

Figure 3 (a) shows a functionalized ring according to an embodiment of the invention, which is being supplied with water through two different channels, each one communicating a fluid sample to a different half of the ring. In this embodiment, water flows through both upper and bottom channels. Therefore, no analyte molecules binding events have occurred. As a consequence, Figure 3 (c) shows the degeneracy of the spectra when in absence of analyte molecules.

Figure 3 (b) shows the functionalized ring of Figure 3 (a) being supplied with a solution of water and molecules through one of the channels, thus showing analyte molecules attached to its perimeter. Accordingly, Figure 3 (d) shows the degeneracy lifting of the spectra when analyte molecules are attached to one half of the ring.

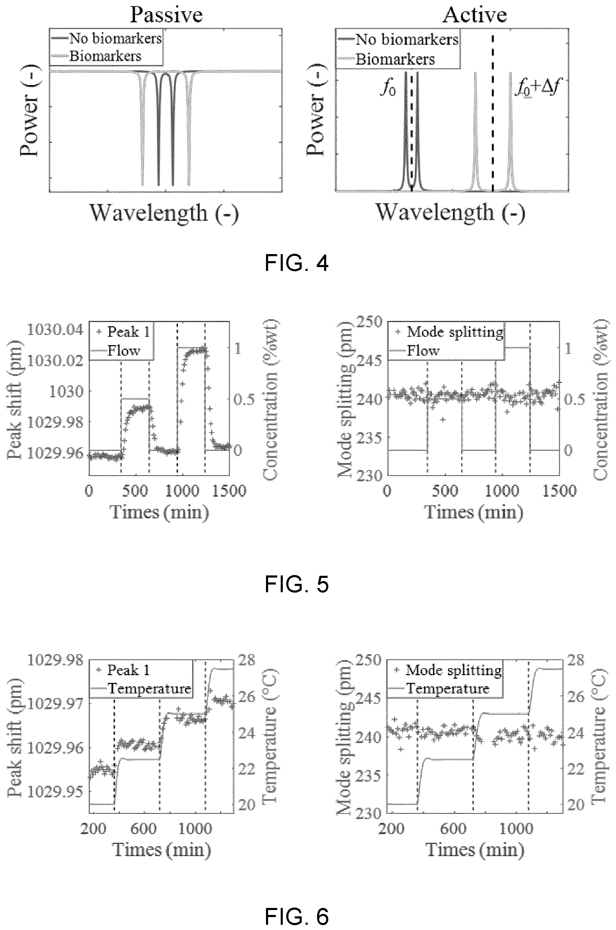

Figure 4 shows the contrast in the emission linewidth between the passive and active embodiments of a self-referenced sensor according to the present invention. Apart from the splitting in the frequency domain (beatnote) due to the presence of the grating, the active case (i.e., resonators with doped material providing a gain medium making them capable of operating as a laser) shows narrower peaks, which leads to higher precision in the detection of the beatnote.

Figure 5 shows the results of an experiment testing the self-referencing capabilities of a sensor according to the invention. On one side, it is shown how the peaks in which the signal is split by the mode splitting (Peak 1 and Peak 2) shift. In this particular case, only Peak 1 is shown as an example. As a function of time, the RIU (Refractive Index Unit) is modified, by increasing every 500s the concentration of the liquid flowing over the sensor. It can be observed that Peak 1 shifts its resonance frequency accordingly.

On the other side, the mode splitting of the sensor, this is, the beatnote variation resulting of subtracting the frequency of Peak 2 to Peak 1, is displayed as a function of time. In contrast with the variation of Peak 1 with the bulk refractive index as a consequence of the increase in the concentration of the liquid flowing over the sensor, said beatnote does not vary.

Figure 6 shows an experiment in which temperature is varied in a similar manner in which RIU was varied in the experiment carried out in figure 5. It can be observed that, whereas Peak 1 shifts subsequently with temperature variation, again, the beatnote remains substantially constant.

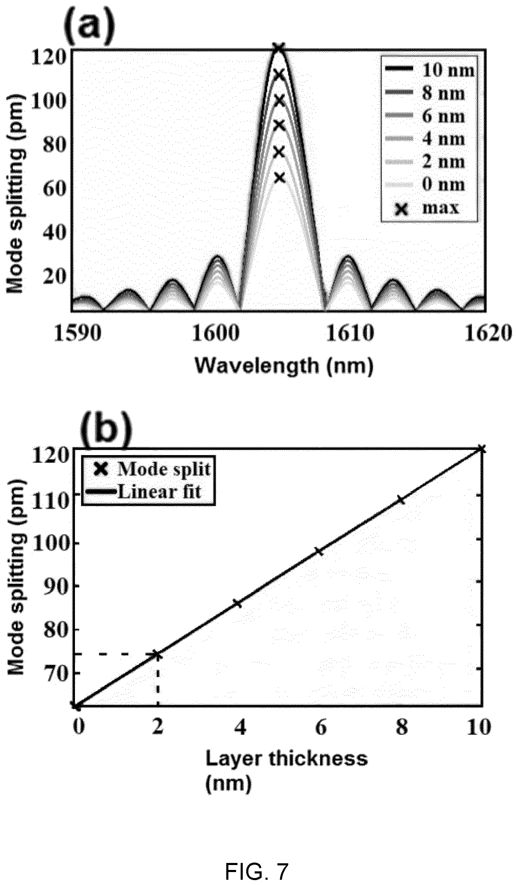

Figure 7 shows the results of different mode-splitting simulations for a resonator according to an embodiment of the present invention. To test the response of the resonator with a grating upon biomarker concentration, several biolayers with different thicknesses have been considered. As it can be seen in the two correlated graphs (a) and (b), the splitting increases monotonously with the number of binding events. This is, as the layer thickness increases as shown with the linear fit in graph (b) (for the selected values of layer thickness (nm) (0, 2, 4, 6, 8, 10)), the mode splitting also increases (having the corresponding peak values shown in graph (a) for the selected values of layer thickness).

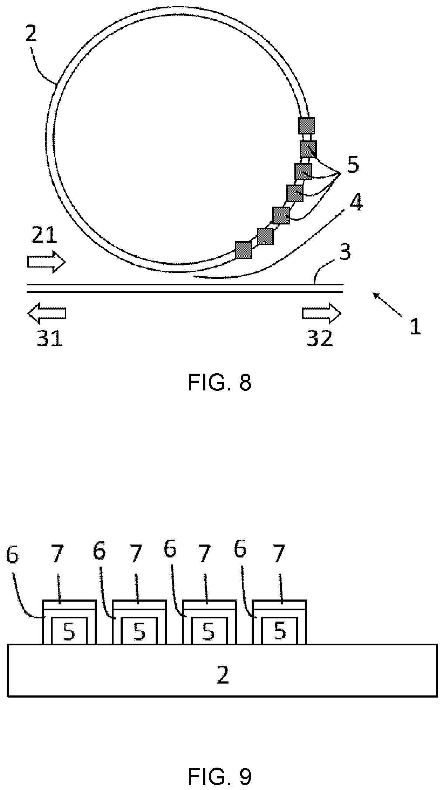

Figure 8 schematically shows a sensor (1) according to an embodiment of the present invention. Specifically, the sensor (1) comprises a micro ring resonator (2) embodied as a looped optical waveguide, namely a circular optical waveguide, and an optical waveguide (3) for coupling with the looped optical waveguide (2). In this embodiment the micro ring resonator (2) has part of the ring perimeter covered by a grating (5). This grating (5) induces a mode splitting that varies upon binding of molecules to predetermined sections of the grating (5). In the figure the grating (5) is schematically represented with grey squares.

In this embodiment the ring resonator (2) is made of $Al_2O_3$ doped with Ytterbium, which allows using the micro ring resonator (2) in an active mode. In the active mode the micro ring resonator (2) operates as a laser, emitting one or several frequencies that are associated to resonator modes within the gain spectrum of the doping material (in this embodiment Ytterbium). Narrowing of the emission linewidth occurs after lasing threshold. A -200 kHz linewidth was measured for a device with output power in the tens of microwatts

There are different ways in which the grating (5) can be provided.

Figure 9 schematically shows an embodiment wherein the grating (5) is generated by adding to a top surface of the micro ring resonator (2) a layer of a different material. In this embodiment the micro ring resonator (2) is made of $Al_2O_3$ and the material of the grating (5) is $SiO_2$. The grating (5) can cover the full circumference of the micro ring resonator (2) of just a portion of it.

Selective functionalization (6) of the grating (5), either by different materials or lift-off process of the functionalization (6), permits the immobilization of analyte receptors and the subsequent attachment of analyte molecules to only part of the grating (5), thus inducing a variation of the induced splitting as a function of the number of analyte molecules attached to the surface of the grating (5). In this embodiment the grating (5) is selectively functionalized with silane (6) to achieve selective immobilization of antibodies on the grating (5). The analyte receptors, which in this embodiment are antibodies, can be immobilized over the full perimeter of the micro ring resonator (2) or over only a part of it. A biolayer (7) is also shown in figure 9, the biolayer (7) comprising the antibodies immobilized on the grating (5) and captured proteins.

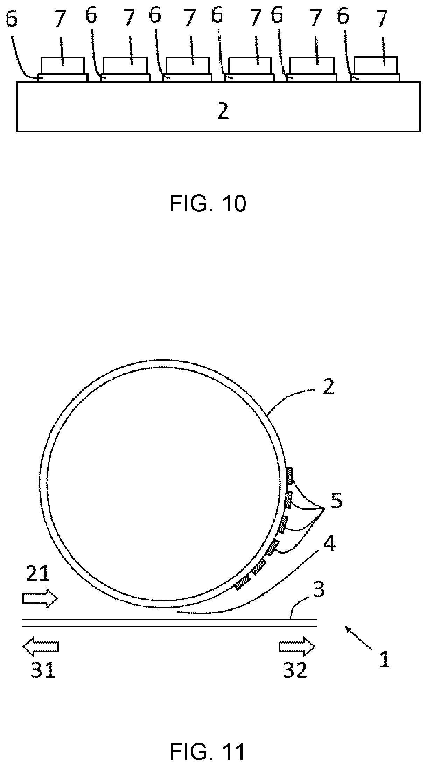

Figure 10 schematically shows an embodiment wherein the grating (5) is provided on the micro ring resonator (2) by directly imprinting analyte receptors onto a top surface of the functionalized (6) micro ring resonator (2). Thus, in this embodiment the functionalization (6) is arranged on the micro ring resonator (2) to form a grating (5). The grating (5) of analyte receptors can cover the full perimeter of the micro ring resonator (2) or only a part of it. In this embodiment the micro ring resonator (2) is made of $Al_2O_3$ and the analyte receptors are antibodies. A biolayer (7) is also shown in figure 10, the biolayer (7) comprising the antibodies immobilized on the grating (5) and captured biomarkers.

In the embodiments of figures 8 to 10 the grating (5) is arranged on the top surface of the micro ring resonator (2). Figure 11 schematically shows a sensor (1) according to an embodiment wherein the grating (5) is arranged on a sidewall over a part of the perimeter of a micro ring resonator (2). Corrugation of the sidewall, corresponding to the grating (5), is represented by rectangles in the figure.

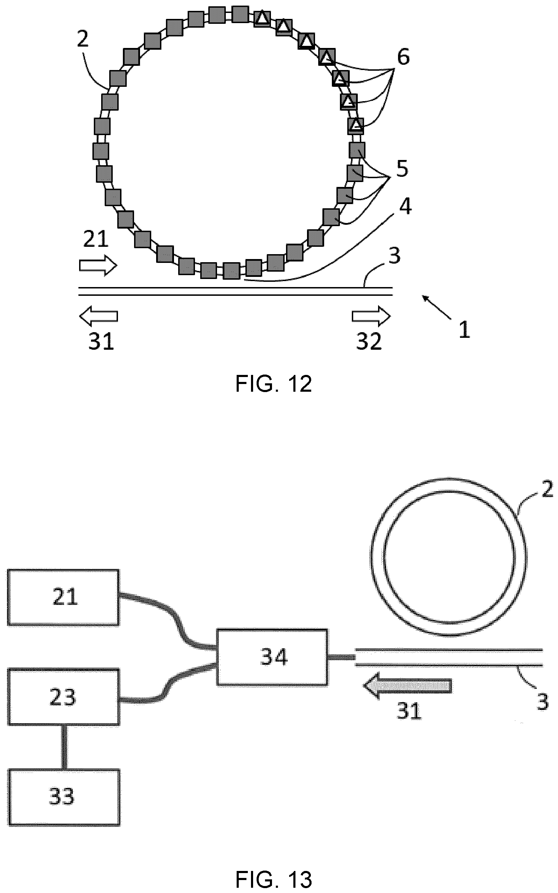

Figure 12 shows a sensor (1) according to an embodiment of the invention. In this embodiment a micro ring resonator (2) has a grating (5) covering its full perimeter. However, only a quarter of the grating (5) is functionalized (6) and covered with analyte receptors.

In figure 12 the grating (5) is represented with grey squares and the functionalization (6) is represented with white triangles.

Figure 13 schematically shows a readout unit for the sensors (1) of figures 8, 11 and 12 when working in an active mode. The readout unit comprises a laser diode pump (21) to optically pump the micro ring/micro disk resonator (2) laser and a fast photodetector (23) followed by an RF spectrum analyser (REF) (33). The system further comprises a Wavelength Division Multiplexing unit (WDM) (34), which enables bidirectional transmission through the straight optical waveguide (3), multiplexing the pump laser (21) and the backward lasing power (31). In an embodiment the photodetector (23) is a photodiode. If the splitting is within or below the GHz range, then conventional electronics can be used to detect the beating between the splitted modes. In this way, the readout unit can be made portable and inexpensive. In figures 8, 11 and 12 the pump laser (21), the backward lasing power (31) and the forward lasing power (32) are schematically represented with arrows. In these figures a straight optical waveguide (3) is also shown, separated from the micro ring resonator (2) by a gap (4), but sufficiently near to allow optical coupling.

Although the embodiment of figure 13 operates in an active mode, in other embodiments the sensors (1) work in a passive mode. In a particular embodiment, if operated as a passive device, during the operation of the sensor (1), the resonator (2) can be excited by a laser with a spectral bandwidth that is large enough to include the two split resonances. In this embodiment the readout unit comprises the laser and a photodetector.

In another particular embodiment of the passive mode, the readout unit comprises a tunable laser along with a photodetector (23) for scanning the resonances of the resonator (2), so that the peak position of the two split resonances can be detected. Even if the embodiments of figures 8, 11 and 12 have been described referring to a micro ring resonator (2), in other embodiments the resonator (2) is embodied as a disk and the above description is also applicable to said disk resonators (2).

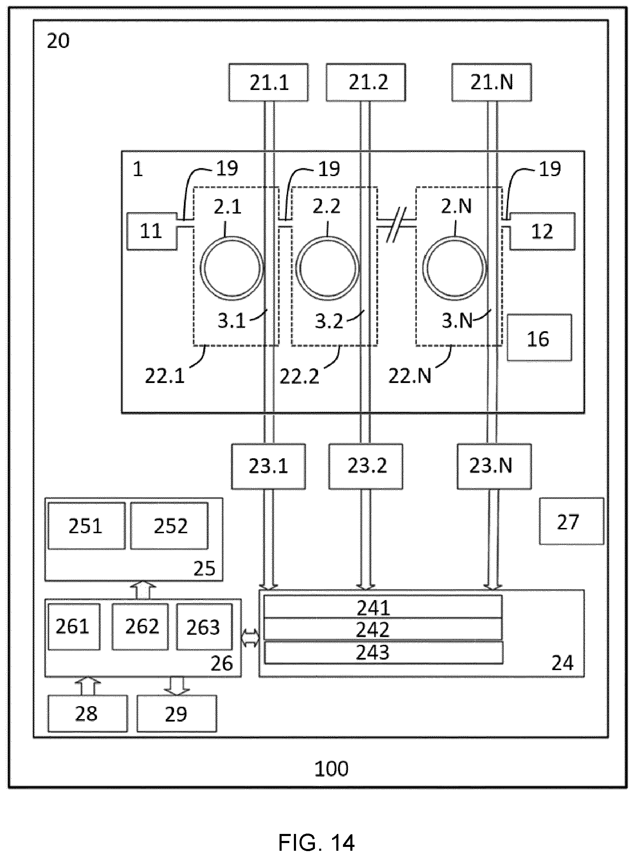

Figure 14 shows an embodiment of a sensing system (100).

The sensing system (100) comprises a sensor (1) and a readout unit (20) configured to receive the sensor (1).

In this embodiment the sensor (1) is a disposable cartridge configured for the detection of a plurality of N biomarkers related to the indication of cancer, preferably N being 4 or more. The sensor (1) has one resonator (2.1, 2.2, ... 2.N) per biomarker, that is, a total number of N resonators (2.1, 2.2, ... 2.N). Each resonator (2.1, 2.2, ... 2.N) is embodied as a looped optical waveguide shaped as a circular ring (2.1, 2.2, ... 2.N). The sensor (1) further comprises N substantially straight optical waveguides (3.1, 3.2, ... 3.N) for optical coupling with the circular rings (2.1, 2.2, ... 2.N). The resonator rings (2.1, 2.2, ... 2.N) comprise a grating (not shown in figure 14) and are functionalized (6) for the immobilization of analyte receptors for the binding of biomarkers associated to the respective cancer type (one type of biomarker per resonator).

The number N of resonators in the sensor (1) provides the number of biomarkers that can be analyzed simultaneously, with one sensor (1) and one fluid sample. A higher number of resonators (2.1, 2.2, ... 2.N) allows more biomarkers to be analyzed simultaneously with one sample, which results in more information and better quality to the user in the form of indication of different cancer classes and/or cancer development stages by different biomarkers with one measurement. Also, it results in an increase in detection probability of a specific cancer class and specific cancer status, by using a higher number of different biomarkers for detecting the same class or specific stage of cancer. On the other side, a higher number of resonators (2.1, 2.2, ... 2.N) would imply that different antibodies for different biomarkers gather biomarker molecules at different speeds, so in case of a higher number of biomarkers it would be more difficult to arrange the optimum time of contact with the sample, therefore making the system (100) calibration more complex. Therefore, a preferred number of resonators (2.1, 2.2, ... 2.N) is between 8 and 16.

The readout unit (20) is the non-disposable part of the sensing system (100). In this embodiment the readout unit (20) contains N optical sources (21.1, 21.2, ... 21.N) and N optical detectors (23.1, 23.2, ... 23.N). The optical sources (21.1, 21.2, ... 21.N) are preferably integrated laser components. In this embodiment the readout unit (20) comprises one optical source (21.1, 21.2, ... 21.N) per resonator (2.1, 2.2, ... 2.N). In another embodiment, the readout unit (20) comprises a single laser source (21.1, 21.2, ... 21.N) and the light is splitted into N number of channels either by a fiber based 1xN splitter or a 1xN splitter integrated on the sensor (1) or on the readout unit (20).

In this embodiment the sensor (1) is provided with an input port (11) for the entry of a fluid sample into the sensor (1) and a container (12) for collecting the fluid sample after the fluid sample passes through the ring resonators (2.1, 2.2, ... 2.N). In this embodiment the sensor (1) comprises a plurality of analysis chambers (22.1, 22.2, ... 22.N) and the resonators (2.1, 2.2, ... 2.N) are arranged such that each analysis chamber (22.1, 22.2, ... 22.N) comprises one resonator (2.1, 2.2, ... 2.N). The sensor (1) has a plurality of openings (19), each opening (19) providing fluid communication between two adjacent analysis chambers (22.1, 22.2, ... 22.N). The inlet port (11), the analysis chambers (22.1, 22.2, ... 22.N) and the container (12) are arranged such that after introduction of the fluid sample through the inlet port (11), the fluid sample passes through the analysis chambers (22.1, 22.2, ... 22.N) until reaching the container (12). The pass of the fluid sample from the inlet port (11) to the container (12) within the sensor (1) can be achieved and/or facilitated by the effect of gravity, by putting the sensor (1) in a position such that the inlet port (11) and the container (12) are substantially placed in a vertical plane, with the inlet port (11) placed at a higher position than the container (12). In an embodiment the openings (19) providing fluid communication between two adjacent analysis chambers (22.1, 22.2, ... 22.N) have different sizes in order to regulate the exposure time of the fluid sample to the analyte receptors in each analysis chamber (22.1, 22.2, ... 22. N). However, other means to regulate the exposure time of the fluid sample to the analyte receptors in each analysis chamber (22.1, 22.2, ... 22.N) may be provided.

The fluid sample may be of human or animal origin. This includes blood, secretion or emulsions made of solid biologic material and is preferably but not necessarily urine. The optical sources (21.1, 21.2, ... 21.N) introduce light into each N optical waveguides (3.1, 3.2, ... 3.N), passing through the resonators (2.1, 2.2, ... 2.N) and initiating resonance in each resonator (2.1, 2.2, ... 2.N). The resonators (2.1, 2.2, ... 2.N) have analyte receptors, specifically antibodies related to the dedicated biomarker, and the presence of the related biomarker causes a shift in optical resonance frequency. N optical waveguides (3.1, 3.2, ... 3.N) approach N optical detectors (23.1, 23.2, ... 23.N), in this particular example laser detector diodes. In this embodiment N optical sources (21.1, 21.2, ... 21.N) and N optical detectors (23.1, 23.2, ... 23.N), are within the readout unit (20). In other embodiments, the optical sources (21.1, 21.2, ... 21.N) and/or the optical detectors (23.1, 23.2, ... 23.N) may be arranged in the sensor (1).

The optical detectors (23.1, 23.2, ... 23.N) are able to detect the level and frequency of optical signals. In the case of passive sensors (1), the laser sources (21.1, 21.2, ... 21.N) are tunable to be able to scan over the resonance. The optical detectors (23.1, 23.2, ... 23.N) are used to detect the backward lasing light (31). In a preferred embodiment the disposable sensor (1) has mechanical means to be connected to the non-disposable readout unit (20) of the sensing system (100) so that active optical alignment is not necessary. The optical detectors (23.1, 23.2, ... 23.N) have a specific dynamic range to detect the minimum and maximum of the expected signal spectrum.

In this embodiment the readout unit (20) contains a signal processing module (24), a communications interface (25) and a supporting electronic unit (26). In this embodiment the readout unit (20) additionally comprises an operation start button (28) and a data processing done indicator (29).

In this embodiment the signal processing module (24) includes a signal conditioning unit (241), an analog to digital conversion (ADC) unit (242) and a digital processing unit (243). The signal processing module (24) is configured to measure the beatnote variation coming from optical detectors (23.1, 23.2, ... 23.N).

The signal conditioning unit (241) contains amplification of the analog signal coming from the optical detectors (23.1, 23.2, ... 23.N), as well as analog filtering. The purpose of the signal conditioning unit (241) is to provide a noise-free analog signal to be adequately captured by the analog to digital conversion unit (242), such that the complete dynamic range of the signal may be correctly covered with the best possible resolution. Signal conditioning unit (241) may have also analog down conversion functionalities, if the realized beatnote variation is too large to be detected by analog digital conversion electronics.

For each dedicated resonator (2.1, 2.2, ... 2.N), the processing results provide information on the beatnote variation, being associated with probability of detection that is further analyzed. For each biomarker, being related to each resonator (2.1, 2.2, ... 2.N), the sensing system (100) has a predefined mapping structure to perform classification of data obtained from the sensor (1). This means that for each biomarker type the sensing system (100) knows the mapping between beatnote variation, detection probability and information on the biomarker concentration. Therefore, the beatnote variation is monitored over time and correlated with the analyte concentration. In an embodiment the mapping results from a previous calibration of the sensing system and/or of the sensor. In an embodiment the mapping is stored in a memory of the readout unit and/or is remotely stored is a separate device accessible from the readout unit.

In an embodiment the analyte receptors attached to the resonators (2.1, 2.2, ... 2.N) are N antibodies configured for the binding of a cancer biomarker, wherein the cancer biomarkers are selected from the following list of biomarkers:

- PSA, for prostate cancer
- Engrailed 2, for prostate cancer
- MSMbeta for prostate cancer
- HAS-1, for bladder cancer:
- HAS-2, for bladder cancer:
- HYAL-3, for bladder cancer:
- Cytokeratin 20 (CK20), for bladder cancer:
- CAIX, for renal cancer:
- Aquaporin1, for renal cancer:
- Adipophilin, for renal cancer:
- IL8, for bladder cancer
- MMP9, for bladder cancer
- MMP10, for bladder cancer
- SERPINA1, for bladder cancer
- VEGFA, for bladder cancer
- ANG, for bladder cancer
- CA9, for bladder cancer
- APOE, for bladder cancer
- SDC1, for bladder cancer
- SERPINE1, for bladder cancer
- HYAL1, for bladder cancer
- CXCL 1, for bladder cancer
- CXCR7, for bladder cancer

- SDF1beta, for bladder cancer

The information acquired from the sensor (1) is classified and provided, preferably together with biofluid sample and sensor (1) identification, to the supporting electronic unit (26). In this embodiment the supporting electronic unit (26) includes Human Machine Interface (HMI) unit (261), a power supply (262) and a controlling unit (263). In the supporting electronic unit (26) the information may be:

- stored on the associated memory, where one of the options can be a removable memory realization like a Secure Digital (SD) card or flash memory;
- provided to the communications interface (25); and/or
- provided to the HMI unit (261).

The HMI unit (261) provides interaction with the user by multimedia or mechanical means, including interaction with a display of the non-disposable readout unit (20), displaying one or more of the following results: biomarker detection, probability of dedicated biomarker detection, cancer class probability detection or indication, time information, environment information such as temperature, disposable part identification, apparatus status, such as battery status, apparatus readiness to perform and/or apparatus activity, using any applicable graphical and/or textual means. The HMI unit (261) can also provide information by electrically generated sound, such as information on sensing system (100) status information and/or on activity results, by using a loudspeaker being part of the readout unit (20). Thus, warnings or status indications like "data analysis process started", "data analysis process completed", "battery low" or "ready to perform", are addressed. Also, mechanical vibrations could be performed as status warnings or status indications like "data analysis process started", "data analysis process completed", "battery low" or "ready to perform".

The controlling unit (263) may be implemented as micro-controller software code or may be integrated as extra software code of the processor unit (24) performing digital signal processing (243), which preferably is a CPU, like an ARM class microprocessor. Alternatively, the controlling unit (263) may be implemented as a unit separate from the digital signal processing unit (243). The controlling unit (263) takes part in the status of sensing system (100), including environment and power status control as well as control of the sensing system's (100) functional blocks. It also interacts with the HMI unit (261). The controlling unit (263) provides information on the presence of disposable sensor (1) inserted in non-disposable readout unit (20), information on the type of disposable sensor (1), as well as on the operation of start button (28) and data processing done indicator (29).

Operation start button (28) is advantageously realized by soft pressure system button technology which allows for good water-proof realization. Operation start button (28) is advantageously realized with an optical indicator system showing in one color the "status done" signal, in another color the "ready for sensor acquisition" signal, and in a third color the "no cartridge attached" signal.

In this embodiment the communications interface (25) comprises a wireless connection interface (251) and a wired connection interface (252). This allows communication from the readout unit (20) to the world outside of the sensing system (100). In a preferred embodiment, the wired connection interface (252) has one or more of the following wired communication means: USB connector, Ethernet connection, CAN based cable connection, LIN Based connector cable connection, SPI wired connection, UART wired connection. The wired connection interface (252) can enable communication to the cloud or to a remote memory on user PC or user network. The wireless connection interface (251) may be realized by a plurality of short range and log range wireless means, including: 2G, 3G, LTE mobile communication interface and/or other long range, cellular, communication methods; wireless LAN, Bluetooth, short range communication methods in ISM bands.

The readout unit (20) may include a memory for the storage of information, such as a cartridge reference number and/or findings of the executed data acquisition and data processing procedures when the sample is analyzed. This may allow for better tracking and system (100) optimization. Also, the readout unit (20) may include a detector, preferably based on RFID technology, for the detection of an identifier (16), preferably a passive RF ID tag, located on the sensor (1).

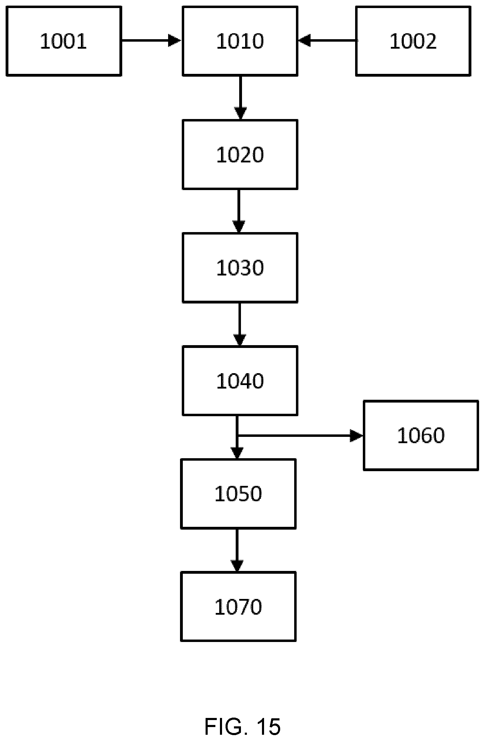

Figure 15 shows a flowchart showing the method of operation of the sensing system (100), the method comprising the following steps from the user perspective:

- Inserting (1010) the sensor (1) in the readout unit (20) of the sensing system (100).
- Putting (1020) a fluid sample in the input port (11) of the sensor (1).
- Initiating (1030) the electronics of the readout unit (20), together with the optical sources (21) and optical detectors (23) by pressing operation start button (28).
- Allowing the biomarker detection to be performed until a visual indication of completed processing is shown (1040) by the data processing done indicator (29) of the readout unit (20).
- Acquiring (1050) from the HMI unit (261) the analysis results, specifically indication of cancer biomarkers presence or absence in the fluid sample, the presence indication being preferably accompanied with a probability indication.
- Optionally, the analysis results and/or details of cancer biomarker signal processing data are made available on an external device like a computer, portable computer, tablet, mobile phone and/or data cloud, the data being transmitted (1060) from the readout unit (20) through the communications interface (25), via wireless (251) and/or wired (252) means. The concentration of biomarkers will be determined from the calibrated sensor (1).
- The user removes (1070) the sensor (1) from the readout unit (20), optionally disposing the remaining fluid sample through an output of the container (12). The sensor (1) may also be disposed and/or recycled.

Optionally, initial operation settings can be obtained (1001) from an external system, such as a data cloud, a computer or a mobile platform, or can be inserted (1002) using the HMI entity (261).

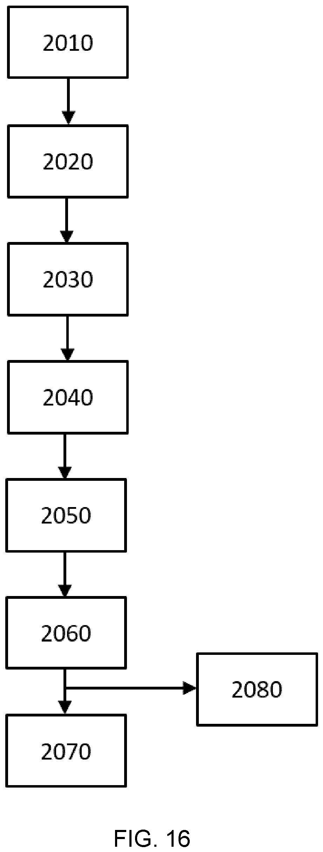

With reference to figure 16, the method of operation of the sensing system (100) comprises the following steps from the functional perspective:

- The optical sources (21.1, 21.2, ... 21.N) generate (2010) optical signal emissions with a dedicated wavelength. The optical sources (21.1, 21.2, ... 21.N) are preferably lasers.
- Fluid sample, influenced by gravity, passes (2020) from the inlet port (11) to the first chamber (22.1), where the first resonator (2.1) is present, and subsequently passes through the other chambers (22.2, ... 22.N) until ending in the container (12).
- The optical waveguides (3.1, 3.2, ... 3.N) are coupled with the resonators (2.1, 2.2, ... 2.N), inducing the resonance processes (2030). The resonators (2.1, 2.2, ... 2.N) have antibodies related to the dedicated biomarker, the antibodies being immobilized on the resonators (2.1, 2.2, ... 2.N) surface.
- The resonators (2.1, 2.2, ... 2.N) interact with the fluid sample entered into the sensor (1). If the fluid sample contains biomarker molecules matching antibodies immobilized on the resonator (2.1, 2.2, ... 2.N), the biomarker molecules will attach selectively to the part of the resonator (2.1, 2.2, ... 2.N) where the antibodies are immobilized, inducing (2040) a variation of the beatnote that is proportional to the concentration of biomarkers in the fluid sample.
- The optical detectors (23.1, 23.2, ... 23.N) detect (2050) the beatnote variation and provide analog signal information.
- The signal processing module (24) performs (2060) sequential analog signal conditioning, analog to digital signal conversion and digital signal processing to detect for each resonator (2.1, 2.2, ... 2.N) the existence of beatnote variation and the value of the beatnote variation.
- HMI unit (261) shows (2070) the analysis results.
- Optionally, the communications interface (25) sends (2080) the analysis results and/or details of cancer biomarker signal processing data to an external device like a computer, portable computer, tablet, mobile phone and/or data cloud, the data being transmitted (2080) from the readout unit (20) through the communications interface (25), via wireless (251) and/or wired (252) means.

In summary, the following two cases can be obtained:

- The optical signal passing by the resonators (2.1, 2.2, ... 2.N) will have no change of beatnote, if fluid sample does not contain dedicated biomarkers.
- In contrast, if the beatnote change is observed, a dedicated biomarker is detected in fluid sample. Evaluating the value of beatnote variation by applying dedicated calibration processing algorithms, combined with duration of fluid sample interchange with the resonators (2.1, 2.2, ... 2.N) will lead to information of relevant biomarker molecule concentration in fluid sample. This biomarker molecule concentration information in fluid sample, indirectly yields information about the probability of relevant cancer detection.

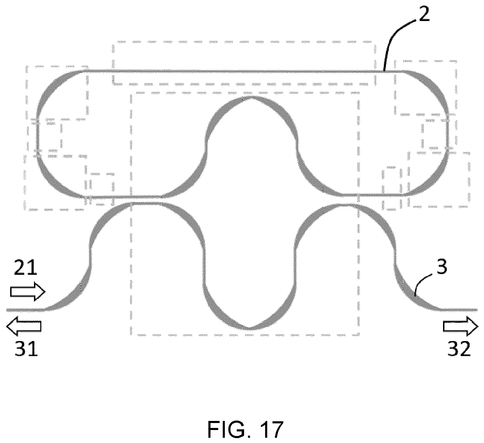

Figure 17 shows an embodiment of the sensor wherein the resonator (2) is shaped as a non-circular loop. Also, a non-straight optical waveguide (3) for coupling with the resonator (2) is shown. The resonator (2) and the waveguide (3) can be designed with different sections, what provides the design process with more flexibility. This is, the shape of the resonator (2) permits engineering of the sensitivity and tolerance for manufacturability errors as well as to tailor the coupling of both pump and signal independently, and to tune the length of the resonator (2), everything without affecting the coupling region. Different sections of the resonator (2) and the waveguide (3) are identified in figure 17 with dash lines.

**Claims**

1. A sensor comprising at least one whispering gallery mode resonator, wherein the resonator comprises a Bragg grating arranged over at least a portion of the perimeter of the resonator and wherein the resonator is selectively functionalized for the attachment of analyte receptors.

**2.** The sensor according to claim 1, wherein the grating is functionalized for the attachment of analyte receptors.

**3.** The sensor according to any of the previous claims, wherein the resonator is made of a first material and the grating is made of a second material different to the first material.

**4.** The sensor according to any of the previous claims, wherein the grating is a grating of analyte receptors directly arranged over a surface of the resonator.

**5.** The sensor according to any of the previous claims, wherein the resonator is made of at least a material selected from $Al_2O_3$, $Si_3N_4$, $SiO_2$, SiOn, $TiO_2$, $Ta_2O_5$, $Te_2O_5$, phosphate glass, $KY(WO_4)_2$, YAG, ZBLAN.

**6.** The sensor according to any of the previous claims, wherein the grating is made of a material comprising at least one of a polymer, preferably PMMA, $SiO_2$, SiOn, SiN or $TiO_2$.

**7.** The sensor according to any of the previous claims, wherein the period of the grating is substantially half the operating wavelength.

**8.** The sensor according to any of the previous claims, wherein the resonator material is doped with a material providing laser gain, preferably a rare-earth material, a semiconductor material, or a transition metal ion doped material.

**9.** The sensor according to any of the previous claims, wherein the sensor is functionalized over only a part of its perimeter, preferably over a quarter of the perimeter or over a half of the perimeter.

**10.** The sensor according to any of the previous claims, wherein:

the resonator is a ring resonator embodied as a looped optical waveguide and the sensor further comprises a coupling mechanism to access the looped optical waveguide, or
the resonator is shaped as a disk and the sensor further comprises a coupling mechanism to access the disk.

**11.** The sensor according to any of the previous claims, wherein the resonator has a closed loop configuration comprising a plurality of sections, each section being configured for coupling with an optical waveguide at a wavelength.

**12.** The sensor according to any of the previous claims, wherein the resonator is fabricated in rare-earth doped $Al_2O_3$, wherein the rare-earth ions provide emission outside the absorption bands of water, the rare-earth ions being preferably selected from the group consisting of $Yb^{3+}$, $Nd^{3+}$, $Er^{3+}$, $Tm^{3+}$, and $Ho^{3+}$.

**13.** The sensor according to any of the previous claims, wherein the sensor comprises a plurality of resonators and a plurality of chambers, wherein the resonators are arranged such that each chamber comprises one resonator, wherein the sensor comprises a plurality of openings, each opening providing fluid communication between two adjacent chambers, and wherein the openings have different size.

**14.** A sensing system comprising:

a sensor according to any of the previous claims, and
a readout unit configured to receive the sensor and comprising aligning means for aligning the sensor.

**15.** The sensing system according to claim 14, further comprising a laser configured to optically pump the at least one resonator when the sensor is received in the readout unit, a photodetector and processing electronics.

**Patentansprüche**

**1.** Sensor, umfassend wenigstens einen Flüstergalerieresonator, wobei der Resonator ein Bragg-Gitter umfasst, das über wenigstens einen Abschnitt des Umfangs des Resonators angeordnet ist, und der Resonator selektiv für die Anbringung von Analytrezeptoren funktionalisiert ist.

**2.** Sensor nach Anspruch 1, bei dem das Gitter für die Anbringung von Analytrezeptoren funktionalisiert ist.

3. Sensor nach einem der vorhergehenden Ansprüche, bei dem der Resonator aus einem ersten Material und das Gitter aus einem zweiten, vom ersten Material verschiedenen Material hergestellt ist.

4. Sensor nach einem der vorhergehenden Ansprüche, bei dem das Gitter ein Gitter aus Analytrezeptoren ist, das direkt über einer Oberfläche des Resonators angeordnet ist.

5. Sensor nach einem der vorhergehenden Ansprüche, bei dem der Resonator aus wenigstens einem Material besteht, das aus $Al_2O_3$, $Si_3N_4$, $SiO_2$, SiOn, $TiO_2$, $Ta_2O_5$, $Te_2O_5$, Phosphatglas, $KY(WO_4)_2$, YAG, ZBLAN ausgewählt ist.

6. Sensor nach einem der vorhergehenden Ansprüche, bei dem das Gitter aus einem Material besteht, das wenigstens eines der folgenden Materialien umfasst: ein Polymer, vorzugsweise PMMA, $SiO_2$, SiOn, SiN oder $TiO_2$.

7. Sensor nach einem der vorhergehenden Ansprüche, bei dem die Periode des Gitters im Wesentlichen die Hälfte der Betriebswellenlänge beträgt.

8. Sensor nach einem der vorhergehenden Ansprüche, bei dem das Resonatormaterial mit einem Material dotiert ist, das eine Laserverstärkung bewirkt, vorzugsweise mit einem Seltenerdmaterial, einem Halbleitermaterial oder einem mit Übergangsmetallionen dotierten Material.

9. Sensor nach einem der vorhergehenden Ansprüche, wobei der Sensor nur über einen Abschnitt seines Umfangs funktionalisiert ist, vorzugsweise über ein Viertel des Umfangs oder über die Hälfte des Umfangs.

10. Sensor nach einem der vorhergehenden Ansprüche, bei dem:

    der Resonator ein Ringresonator ist, der als geschlungener Lichtwellenleiter ausgeführt ist, und der Sensor außerdem einen Kopplungsmechanismus für den Zugang zu dem geschlungenen Lichtwellenleiter umfasst, oder
    der Resonator als Scheibe geformt ist und der Sensor außerdem einen Kopplungsmechanismus für den Zugriff auf die Scheibe umfasst.

11. Sensor nach einem der vorhergehenden Ansprüche, bei dem der Resonator eine geschlossene Schleifenanordnung mit einer Vielzahl von Abschnitten aufweist, wobei jeder Abschnitt für die Kopplung mit einem optischen Wellenleiter bei einer Wellenlänge konfiguriert ist.

12. Sensor nach einem der vorhergehenden Ansprüche, bei dem der Resonator aus mit Seltenerden dotiertem $Al_2O_3$ hergestellt ist, wobei die Seltenerdenionen für eine Emission außerhalb der Absorptionsbanden von Wasser sorgen und die Seltenerdenionen vorzugsweise aus der Gruppe bestehend aus $Yb_{3+}$, $Nd^{3+}$, Er3+, Tm3+ und $Ho^{3+}$ ausgewählt sind.

13. Sensor nach einem der vorhergehenden Ansprüche, wobei der Sensor eine Vielzahl von Resonatoren und eine Vielzahl von Kammern umfasst, wobei die Resonatoren so angeordnet sind, dass jede Kammer einen Resonator umfasst, wobei der Sensor eine Vielzahl von Öffnungen umfasst, wobei jede Öffnung eine Fluidverbindung zwischen zwei benachbarten Kammern herstellt und die Öffnungen eine unterschiedliche Größe aufweisen.

14. Erfassungssystem, umfassend:

    einen Sensor nach einem der vorhergehenden Ansprüche, und
    eine Ausleseeinheit, die dazu eingerichtet ist, den Sensor aufzunehmen, und die Ausrichteinrichtungen zum Ausrichten des Sensors umfasst.

15. Erfassungssystem nach Anspruch 14, weiterhin umfassend einen Laser, der dazu eingerichtet ist, den wenigstens einen Resonator optisch zu pumpen, wenn der Sensor in der Ausleseeinheit empfangen wird, einen Fotodetektor und eine Verarbeitungselektronik.

**Revendications**

1. Un capteur comprenant au moins un résonateur à mode galerie, le résonateur comprenant un réseau de Bragg agencé sur au moins une partie du périmètre du résonateur et le résonateur étant sélectivement fonctionnalisé pour la fixation de récepteurs d'analyte.

2. Le capteur selon la revendication 1, dans lequel le réseau est fonctionnalisé pour la fixation de récepteurs d'analytes.

3. Le capteur selon l'une quelconque des revendications précédentes, dans lequel le résonateur est réalisé en un premier matériau et le réseau est réalisé en un deuxième matériau différent du premier matériau.

4. Le capteur selon l'une quelconque des revendications précédentes, dans lequel le réseau est un réseau de récepteurs d'analyte agencé directement sur une surface du résonateur.

5. Le capteur selon l'une quelconque des revendications précédentes, dans lequel le résonateur est constitué d'au moins un matériau choisi parmi $Al_2O_3$, $Si_3N_4$, $SiO_2$, SiOn, $TiO_2$, $Ta_2O_5$, $Te_2O_5$, verre phosphate, $KY(WO_4)_2$, YAG, ZBLAN.

6. Le capteur selon l'une quelconque des revendications précédentes, dans lequel le réseau est réalisé en un matériau comprenant au moins un parmi un polymère, de préférence le PMMA, le $SiO_2$, le SiOn, le SiN ou le $TiO_2$.

7. Le capteur selon l'une quelconque des revendications précédentes, dans lequel la période du réseau est sensiblement la moitié de la longueur d'onde de fonctionnement.

8. Le capteur selon l'une quelconque des revendications précédentes, dans lequel le matériau résonateur est dopé avec un matériau apportant un gain laser, de préférence un matériau de terre rare, un matériau semiconducteur ou un matériau dopé aux ions de métal de transition.

9. Le capteur selon l'une quelconque des revendications précédentes, dans lequel le capteur est fonctionnalisé sur une partie seulement de son périmètre, de préférence sur un quart de périmètre ou sur une moitié de périmètre.

10. Le capteur selon l'une quelconque des revendications précédentes, dans lequel :

   le résonateur est un résonateur en anneau réalisé sous la forme d'un guide d'ondes optique en boucle et le capteur comprend en outre un mécanisme de liaison pour accéder au guide d'ondes optique en boucle, ou le résonateur a la forme d'un disque et le capteur comprend en outre un mécanisme de liaison pour accéder au disque.

11. Le capteur selon l'une quelconque des revendications précédentes, dans lequel le résonateur a une configuration en boucle fermée comprenant une pluralité de sections, chaque section étant configurée pour se relier avec un guide d'onde optique à une longueur d'onde.

12. Le capteur selon l'une quelconque des revendications précédentes, dans lequel le résonateur est fabriqué en $Al_2O_3$ dopé aux terres rares, les ions de terres rares assurant une émission en dehors des bandes d'absorption de l'eau, les ions de terres rares étant de préférence choisis parmi le groupe composé de $Yb^{3+}$, $Nd^{3+}$, $Er^{3+}$, $Tm^{3+}$ et $Ho^{3+}$.

13. Le capteur selon l'une quelconque des revendications précédentes, dans lequel le capteur comprend une pluralité de résonateurs et une pluralité de chambres, les résonateurs étant agencés de sorte que chaque chambre comprend un résonateur, le capteur comprenant une pluralité d'ouvertures, chaque ouverture assurant une communication fluidique entre deux chambres adjacentes, et les ouvertures ayant des tailles différentes.

14. Un système de détection comprenant :

   un capteur selon l'une quelconque des revendications précédentes, et une unité de lecture configurée pour recevoir le capteur et comprenant des moyens d'alignement pour aligner le capteur.

15. Le système de détection selon la revendication 14, comprenant en outre un laser configuré pour pomper optiquement ledit au moins un résonateur lorsque le capteur est reçu dans l'unité de lecture, un photodétecteur et une électronique de traitement.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

```
┌──────────┐       ┌──────────┐       ┌──────────┐
│   1001   │──────▶│   1010   │◀──────│   1002   │
└──────────┘       └──────────┘       └──────────┘
                        │
                        ▼
                   ┌──────────┐
                   │   1020   │
                   └──────────┘
                        │
                        ▼
                   ┌──────────┐
                   │   1030   │
                   └──────────┘
                        │
                        ▼
                   ┌──────────┐       ┌──────────┐
                   │   1040   │──────▶│   1060   │
                   └──────────┘       └──────────┘
                        │
                        ▼
                   ┌──────────┐
                   │   1050   │
                   └──────────┘
                        │
                        ▼
                   ┌──────────┐
                   │   1070   │
                   └──────────┘
```

FIG. 15

FIG. 16

FIG. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7796262 B

**Non-patent literature cited in the description**

- **SASI MUDUMBA et al.** Photonic ring resonance is a versatile platform for performing multiplex immunoassays in real time. *Journal of Immunological Methods,* 2017, vol. 448, 34-43, https://doi.org/10.1016/j.jim.2017.05.005
- **HE, LINA et al.** Detecting single viruses and nanoparticles using whispering gallery microlasers. *Nature nanotechnology,* 2011, vol. 6 (7), 428
- Silicon photonic MZI sensor array employing on-chip wavelength multiplexing. **MUELLNER P et al.** OPTICAL AND QUANTUM ELECTRONICS. KLUWER ACADEMIC PUBLISHERS, 05 February 2012, vol. 44, 557-562